# EUROPEAN PATENT APPLICATION

(11) **EP 4 242 323 A1**
(43) Date of publication of application: **13.09.2023**
(21) Application number: 21888735.4
(22) Date of filing: 08.11.2021
(51) Int. Cl.: C12Q 1/6806, C12Q 1/6853

(54) **METHOD FOR PRODUCING MIRNA LIBRARIES FOR MASSIVE PARALLEL SEQUENCING**

(30) Priority: 06.11.2020 ES 202031119
(71) Applicant: Universidad de Granada, 18071 Granada (ES); Servicio Andaluz de Salud, 41071 Sevilla (ES); López-Viota Gallardo, Julián, 18008 Granada (ES)
(72) Inventor: CARAZO GALLEGO, Ángel, 41071 Sevilla (ES); REDRUELLO ROMERO, Anaïs, 18071 Granada (ES); LÓPEZ PÉREZ, David, 18071 Granada (ES); LEÓN LÓPEZ, Josefa, 41071 Sevilla (ES); MORALES SANTANA, Sonia, 41071 Sevilla (ES); VERBENI, Michela, 18071 Granada (ES); LÓPEZ-VIOTA GALLARDO, Julián, 18008 Granada (ES)
(74) Representative: Hoffmann Eitle
(86) International application number: PCT/ES2021/070804
(87) International publication number: WO 2022/096768

(57) **Abstract**

The present invention relates to a method for producing miRNA libraries for massive parallel sequencing by applying nanotechnology which allows biases to be reduced and efficiency to be increased.

## Description

The present invention is comprised in the field of molecular biology and nanotechnology and relates to a method for producing miRNA libraries for massive parallel sequencing by applying nanotechnology for reducing biases, increasing efficiency, and reducing costs.

### PRIOR ART

Eukaryotic cells (and some viruses) produce small non-coding RNA molecules (19 to 25 nucleotides in their mature forms) which regulate the expression of a number of genes. In humans, it has been estimated that 30% of the genome is regulated by microRNAs. microRNAs mainly act on messenger RNAs in the cytoplasm, recognizing specific sequences of UTRs (untranslated regions) through which they reduce the frequency of translation and the half-life of the messenger. Molecular functioning of a certain complexity requires the involvement of protein structures in the cytoplasm (such as the RNA-induced silencing complex, RISC).

miRNAs play a relevant role in processes as important as cell proliferation, apoptosis, differentiation, or energy metabolism. miRNA biogenesis is subjected to strict spatial and temporal control. miRNA deregulation is associated with most chronic pathological processes in humans (including cancer, diabetes, endothelial dysfunction, and neurodegenerative diseases). The stability of miRNA in blood and its relevance in chronic pathological processes suggest that markers indicating the presence, degree, and prognosis of a disease could be found within the population of circulating miRNAs. In this sense, there is an important field of research in cancer (included in the concept of liquid biopsy) and, developed to a lesser extent, in type II diabetes and neurodegenerative diseases.

There are unresolved methodological barriers which limit miRNA analysis, as well as the existence of biases not taken into consideration that lead to the publication of contradicting results and the lack of reproducibility of many studies. Methodological problems originate from two characteristics of miRNAs:
- **Chemical nature.** An extremely small size which lacks common structures (such as the poly(A) tail of messenger RNAs). The chemical nature represents a considerable challenge which conditions the methodology at all levels (purification, reverse transcription, amplification, and ligation reaction).
- **Low concentration.** miRNAs are capable of developing their biological function (suppressing the expression of specific messenger RNAs) at very low concentrations. Accordingly, they are a minority population within the cellular RNA pool. This problem is exacerbated in circulating miRNA studies in which work is often performed with amounts that are much lower than those obtained in tissue.

There are three methodological groups to address miRNA analysis: (1) specific miRNA sequence analysis by means of RT-PCR (reverse transcription - PCR), (2) microarray hybridization, and (3) miRNA-seq (massive parallel sequencing).

The study of circulating miRNAs as cancer biomarkers presents additional challenges. Blood concentrations are much lower than in tumor tissue, but furthermore, the proportion of circulating miRNA originating from neoplastic cells may vary greatly since it depends on the volume of the tumor mass and on the stage of the cancer. Moreover, certain miRNA species may originate from exosomes released by minority neoplastic populations that are, however, of great relevance (such as tumor-initiating cells or cancer stem cells).

Massive parallel miRNA sequencing (miRNA-seq) should be powerful enough to successfully address the challenges of circulating miRNA. However, results are not reproducible between the different methodologies and platforms and worsen when applied to the peculiarities of circulating miRNA. Accordingly, the "classic and obsolete" microarray is still the method of choice for massive miRNA sequence analysis.

The problems of reproducibility do not originate from massive sequencing *per se*, but rather from the level of massive sequencing library (miRNA-seq library) production.

There are several methods to produce miRNA-seq libraries. All these methods use one- or two-step ligation reactions. The ligation reaction introduces biases given that the probability of attaching two molecules (DNA or RNA) depends on their sequences. Accordingly, when producing libraries by means of ligation reactions, a significant alteration of relative frequencies may happen within the population of molecules to be sequenced (overestimating certain sequences and underestimating others). Ligation bias is a yet-to-be-resolved methodological problem, the error of which is very hard to quantify.

Furthermore, in miRNA, biases associated with the ligation reaction increase considerably for two reasons:
1. Increased sampling error. Ligation reactions have very low efficiencies which, along with the low miRNA concentration (particularly in blood), may complicate the quantification of miRNA variants having a lower frequency.
2. Alteration of ligation probability. The influence of the sequence on ligation probability increases considerably in small molecules (RNA or DNA). However, this phenomenon is furthermore of special relevance when RNA molecules are ligated, probably due to the acquisition of different secondary structures which modulate the reaction rate.

Therefore, it is necessary to develop powerful techniques to perform an accurate genetic analysis, and these techniques must be accurate enough so that circulating miRNA can reflect, through its composition, incipient tumors or tumor subpopulations of clinical relevance.

### DESCRIPTION OF THE FIGURES

**Figure 1****.** Elongation of single-stranded strands covalently attached at the 5' end to a magnetic nanoparticle.
**Figure 2****.** Description of the colloidal tool used in the production of miRNA-seq libraries.
**Figure 3****.** Specific capturing of the genetic material on the surface of the colloidal tools.
**Figure 4****.** Reverse transcription on the particles.
**Figure 5****.** Blocking of the particle.
**Figure 6****.** Elongation of the second adapter
**Figure 7****.** Completion of the library.
**Figure 8****.** Agarose gel electrophoresis showing the size of the miRNA libraries. Lane 1, size standard (the two lower bands correspond to sizes of 100 and 200 bp). Lanes 2 and 3, miRNA libraries (from synthetic RNA sequences). Lane 4, blank (the entire process has been followed in parallel, but without adding RNA).
**Figure 9****.** This figure shows 3 sequences which are part of Example 1, in which sequences from the pGEM plasmid which contains a miRNA-seq library inserted in its polylinker site are shown shaded; sequences of the two massive sequencing adapters of the Ion S5 platform are shown in blue; and the synthetic miRNA cDNA is shown in bold.

### DESCRIPTION OF THE INVENTION

The authors of the present invention have developed a new methodology to create miRNA libraries. The new methodology applies nanotechnology to the process, which allows adding (actually elongating) adapters by means of DNA polymerase, avoiding ligation reaction and problems associated therewith (reduced efficiency and biases).

### METHOD OF THE INVENTION

Therefore, a first aspect of the invention relates to a method for obtaining a massive sequencing library with the complementary DNA, cDNA, of a population of miRNAs of interest, which comprises:
a) Capturing the miRNAs of interest on magnetic particles attached to oligonucleotides bearing in their 3' half a purine polynucleotide sequence of between 18 and 20 bases, preferably poly(T) of between 18 and 20 thymines, and in their 5' half the sequence of one of the massive sequencing adapters, the oligonucleotides being attached to the surface of the magnetic particles by means of a covalent bond at the 5' end thereof, by means of a process which comprises treating the population of miRNAs with a poly(purine) polymerase, preferably poly(A) polymerase, such that they acquire a 3' end of between 20 and 30 adenine nucleotides (RNA tailing);
b) Carrying out a reverse transcription reaction;
c) Performing alkaline washing on the particles to remove the substrates of the reverse transcription reaction (dehybridizing the miRNAs and leaving the complementary sequence thereof at the ends of the oligonucleotides) and removing any nucleic acid molecule not covalently attached to the magnetic particles;
d) Blocking the oligonucleotides (attached to the magnetic particles) which have not acquired a cDNA sequence at the 3' end by adding a terminator nucleotide;
e) Performing an elongation of the DNA attached to the particles of the 3' end with a nucleotide tail, preferably a guanine (polyG) tail;
f) Elongating the second massive sequencing adapter at the nucleotide tail, preferably polyG tail, by means of an elongation template consisting of:
   - at the 5' end, a tail of 20 cytosines;
   - at the opposite end, a sequence complementary to the second massive sequencing adapter;
   with the sequence of the miRNAs of interest being arranged between the two adapters;
g) Performing a polymerization reaction, preferably a standard PCR, on the DNA using primers specific for the ends of the two adapters.

Step (a) consists of the specific capturing of the genetic material on the surface of the colloidal tools.

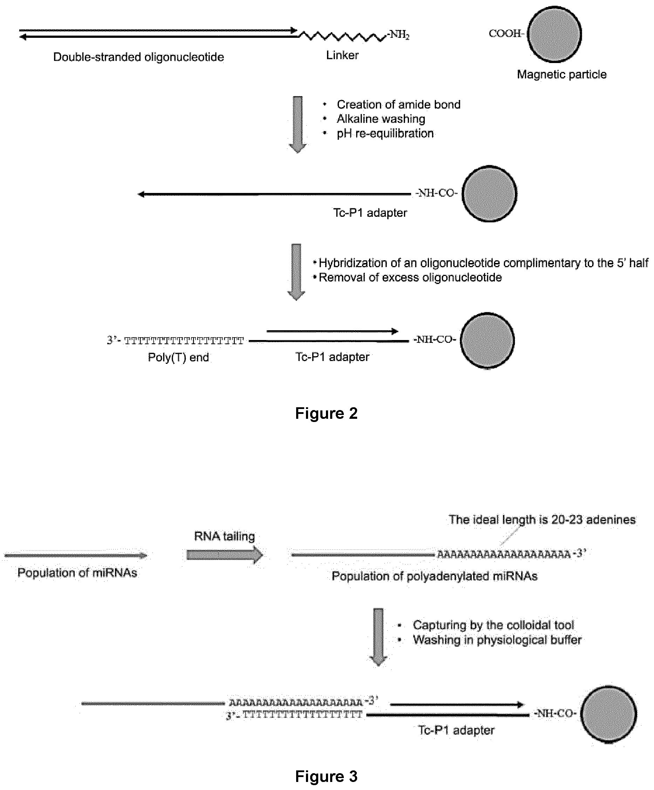

The particle bears on its surface an oligonucleotide covalently attached at its 5' end (in the example of **Figure 2****,** by means of an amide bond). It is advisable to use a linker of 10-20 carbons (-CH2-)n between the amide bond and the oligonucleotide, for the purpose of minimizing steric hindrances on the surface of the particle. The optimal density of the oligonucleotide per unit of particle should be fine-tuned for each magnetic particle-oligonucleotide combination. In the example described herein, the density was 0.5 nM (nanomoles, nMoles) of oligonucleotide per mg of particle. In the example, the amide bond was created by incubating oligonucleotides and particles for 12-14 hours under stochastic stirring in a 250 mM solution (micromoles, mMoles) of 3-(dimethylaminopropyl)-N'-ethylcarbodiimide (EDAC), 1 M of NaCl, and 100 mM MES buffer at pH 5.

Therefore, in a preferred embodiment of this aspect of the invention, the magnetic particle is characterized in that:
I) it has a magnetic core,
II) it has a surface coated with organic compounds with exposed acidic groups that provide them with a negative charge,
III) it is stable at alkaline and acidic PH, within a wide range between pH 2 and 14
IV) it has a low sedimentation coefficient and reduced aggregation,
V) it has a size of between 100 nm (nanometers) and 2000 nm, preferably between 700 nm and 1500 nm, and more preferably of about 800 nm,
VI) it does not inhibit Taq polymerase and can be used in PCR reactions, and
VII) is stable at temperatures up to 100°C.

Ideally, the size of the poly(A) tail added to the 3' end of the miRNAs is of 20-30 nucleotides. The reaction time and the amount of enzyme can be adjusted so that the poly(A) tail is in the desired range. To complete the reaction, heating for 10 minutes at 65°C is sufficient to denature the enzyme.

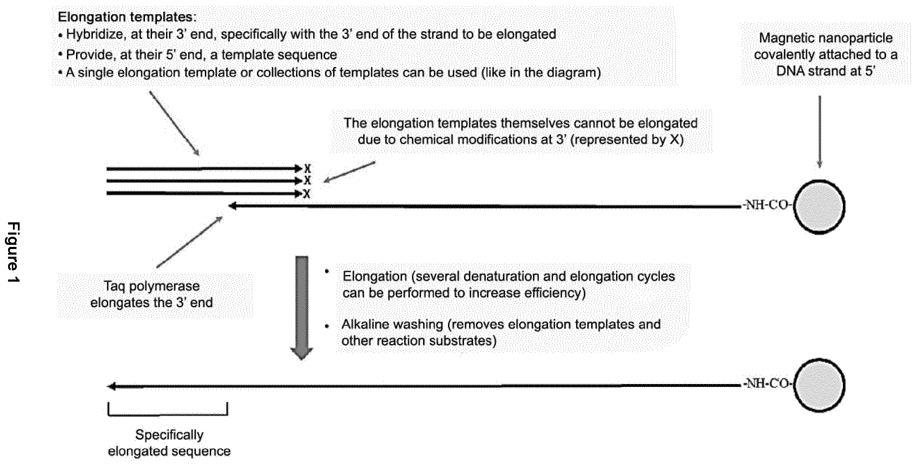

Once the tailing reaction is performed, colloidal tools which capture the population of miRNAs as a result of a single-stranded sequence of (ideally between 18 and 20) thymines are added. The oligonucleotide (DNA) attached to the particle bears, in its 3' half, the poly(T) sequence and, in its 5' half, the sequence of one of the massive sequencing adapters (Figure 1). Said oligonucleotide is attached at its 5' end to the surface of the particles by means of an amide-type covalent bond.

The colloidal tools can capture the miRNAs in the same buffer in which the tailing reaction was performed (i.e., the reaction product does not need to be purified by means of chemical methods). Capturing (by means of hybridization between the poly(A) tail of the miRNAs and the poly(T) sequence of the particles) is performed for 2-4 hours under stochastic stirring and at a temperature of 55°C.

In the example of the invention, after creating the amide bond, the magnetic particles settle on a magnet, washed 2 times with 200 mM NaOH, and incubated in the same alkaline solution for 30 minutes. The sudden increase in pH caused by the NaOH solution has two functions:
- To cause the oligonucleotides covalently attached to the particles to be in a single-stranded form (the non-covalently attached complementary strand is removed during washing).
- To remove unwanted reaction products (acylisourea esters) from the surface of the particle and to restore carboxyl groups in those radicals that have not formed an amide bond with the oligonucleotides.

Once the alkaline incubation is performed, pH is re-equilibrated by means of two washings in 100 mM Tris-HCl buffer at pH 7.4 and the particles are resuspended in 10 mM Tris-HCl buffer at pH 7.4.

In the example of the invention, the sequence of the oligonucleotide covalently attached to the particle consists of:
- In its 3' half, 18 thymines.
- In its 5' half, the sequence of one of the massive sequencing adapters (in the example, Tc-P1 of the Ion S5 platform).

Lastly, the particle is hybridized with a complementary oligonucleotide of the Tc-P1 sequence (in a suitable buffer) and washed in 10 mM Tris-HCl at pH 7.4 to remove excess non-hybridized oligonucleotide. This optional step improves capturing efficiency.

Figure 3 depicts the miRNA tailing reaction and the specific capturing by the colloidal tools.

In step (b) or reverse transcription on the particles, the attachment between the oligonucleotide of the particles and the population of miRNAs is used for priming the reverse transcription reaction. For this reason, it is advisable for the length of the poly(T) tail of the oligonucleotide attached to the particles to be shorter than the poly(A) tail of the miRNAs (which thereby ensures that the 3' ends of the oligonucleotide of the particles remain hybridized to the poly(A) tail of the miRNAs, increasing reverse transcription efficiency).

The particles attached to miRNAs are resuspended in a reverse transcription reaction medium (any commercial reverse transcription kit can be used) which is (conventionally) developed by means of a 30-60 minute incubation at 42°C. Right before adding the reverse transcriptase, it is advisable to perform heating at 70-80°C for 5-10 minutes in order to remove secondary RNA structures that may reduce reverse transcription efficiency.

After the reverse transcription reaction, alkaline washing is performed on the particles (step (c)), and this washing has two functions:
- To remove the substrates of the reverse transcription reaction.
- To remove any nucleic acid molecule not covalently attached to the particles.

As a result, the magnetic particles have covalently attached thereto at 5' single-stranded DNA molecules bearing:
- At 5', the sequence of one of the massive sequencing adapters (Tc-P1 of Ion S5 in the example shown) followed by a poly(T) sequence.
- At 3', the copy DNA (cDNA) of the population of miRNAs.

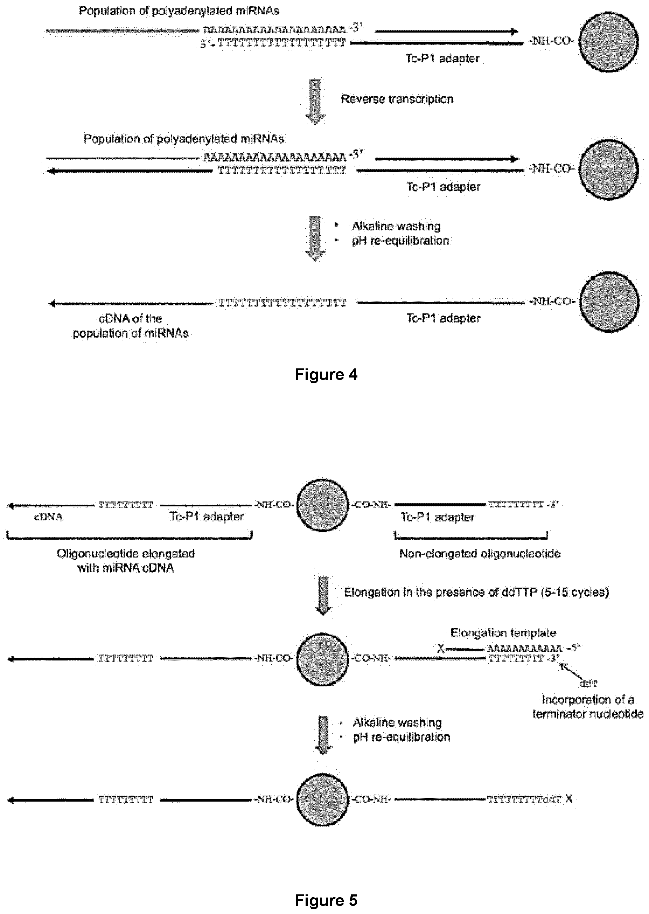

**Figure 4** shows the reverse transcription reaction and the result of alkaline washing.

In step (d), blocking of the particle, the oligonucleotides attached to the colloidal tool must be in excess in order to increase miRNA capturing and reverse transcription efficiency. Accordingly, after steps (a) - (c), a high proportion of oligonucleotides does not undergo elongation (does not acquire a cDNA sequence at 3'). These oligonucleotides without elongation must be blocked so that they do not interfere with the final steps of the process. Blocking of the particle is an essential requirement.

Blocking is performed by selectively adding a terminator nucleotide (dideoxy-thymine) at the 3' end of the oligonucleotides which have not acquired cDNA sequences. To that end, the particles are resuspended in a medium with PCR reaction buffer, Taq-polymerase (any Taq-polymerase or another commercial thermostable DNA polymerase can be used), an oligonucleotide (which was referred to as an elongation template), and dideoxy-thymine-triphosphate (ddTTP) at a concentration of about 0.2 mM.

Dideoxynucleotides (such as dideoxy-thymine) lack a 3' hydroxyl group, and accordingly are incapable of continuously incorporating new nucleotides by means of a phosphodiester bond (which is the basis for Sanger sequencing, for example).

The elongation template consists of:
- At its 5' end, a tail of 21 adenines (in any case, a number greater than the thymine tail of the oligonucleotides of the colloidal tools).
- At the opposite end, a sequence complementary to the massive sequencing adapter.
- Optionally, a dideoxynucleotide at the 3' end which prevents the elongation of the template itself.

By means of this elongation template, a dideoxy-thymine is incorporated only in those oligonucleotides of the particle which have not acquired a cDNA sequence.

The blocking reaction is performed following a protocol consisting of several cycles:
- Denaturation at 95°C
- Hybridization of the elongation template (about 60°C)
- Elongation of the 3' ends hybridized to the elongation template (at the optimal DNA polymerase temperature, normally 72-74°C)

The proportion of elongated molecules can be increased by performing several cycles (5-15 cycles), reaching practically 100%. This is why the 3' ends of the elongation templates are inactivated (by means of a dideoxynucleotide or another terminator nucleotide). This is to ensure that elongation can only proceed from the 3' ends of the DNA strands covalently attached to the particle.

Finally, two alkaline washings of the particle are performed, followed by pH re-equilibration, and resuspension in buffer at pH 7.4. This alkaline washing removes the blocking reaction medium and the elongation template used to specifically block oligonucleotides without a cDNA sequence (step (d)) and only leaves single-stranded strands covalently attached at 5' on the particle.

The process of blocking non-elongated oligonucleotides of the particle is shown in Figure 5.

Elongation of the second adapter is performed in steps (e) and (f). By means of a terminal transferase, a DNA tailing reaction which adds a guanine (poly(G)) tail to the 3' ends (which can be elongated) of the single-stranded DNA attached to the particles is performed. Unlike RNA tailing (step (a)), DNA tailing can add any type of nucleotide to the 3' end of (both single- and double-stranded) DNA molecules. DNA tailing reaction, performed in the presence of only dGTP, adds a poly(G) tail.

For DNA tailing reaction, particles are resuspended in the presence of the suitable reaction medium (any commercial terminal transferase is applicable) and 0.2 mM dGTP. Ideally, the size of the poly(G) tail added to the 3' end of the DNA attached to the particles is of 15-20 nucleotides, never exceeding 20. The reaction time and the amount of enzyme can be adjusted so that the poly(G) tail is within the desired range. To complete the reaction, heating for 10 minutes at 65°C is sufficient to denature the enzyme.

After DNA tailing, magnetic particles are washed twice in a suitable buffer (with PBS, TBS, or another similar buffer) in order to remove the substrates of the reaction.

The poly(G) tail allows elongating the second massive sequencing adapter by means of an elongation template which specifically hybridizes with the poly (G) tail. In this example, this second elongation template consists of:
- At its 5' end, a tail of 20 cytosines (for which reason it is advisable for the poly(G) tail added during the tailing reaction to not exceed 20).
- At the opposite end, a sequence complementary to the second massive sequencing adapter. In this example, adapter A (of Ion S5), the S5 Key sequence, and a sample identifier sequence (barcode 1 of S5) are added.
- Optionally, a dideoxynucleotide at the 3' end which prevents the elongation of the template itself.

The elongation reaction is performed by means of the same method described in step (b), with the exception that instead of dideoxy-thymine-triphosphate, a mixture of the 4 triphosphate nucleotides (dATP, dTTP, dGTP, dCTP) is added at 0.2 mM. Two alkaline washings (and pH re-equilibration) are then performed to remove any remaining reaction medium and DNA strands not covalently attached to the particles.

These alkaline washings remove the elongation reaction medium and the elongation template used to add the sequence of the second adapter, leaving only single-stranded strands covalently attached at 5' on the particle.

Tailing with guanines which hybridize with the poly(C) tail of an elongation template has been shown in the example. The design of the present invention is not limited to this combination and other complementary nucleotide ends can be used.

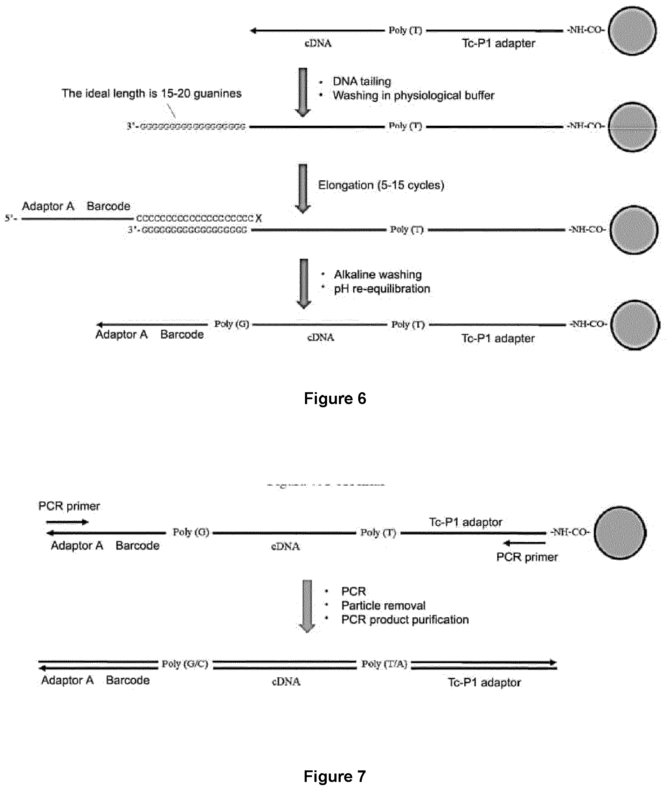

The process of elongating the second adapter is shown in Figure 6.

The library is completed in step (g). For completion, a standard PCR is performed with the particles and using primers specific for the ends of the two adapters. The particle is removed (causing it to settle on a magnet) and the PCR product is purified (Figure 7). In the case of libraries produced from tissue RNA in which larger RNAs (ribosomal and messenger RNAs) have not been removed, it is necessary to perform a selective purification of sizes (below 200 bp). There are several kits on the market which purify DNA based on size, although alternative techniques based on purification after electrophoresis separation can be used.

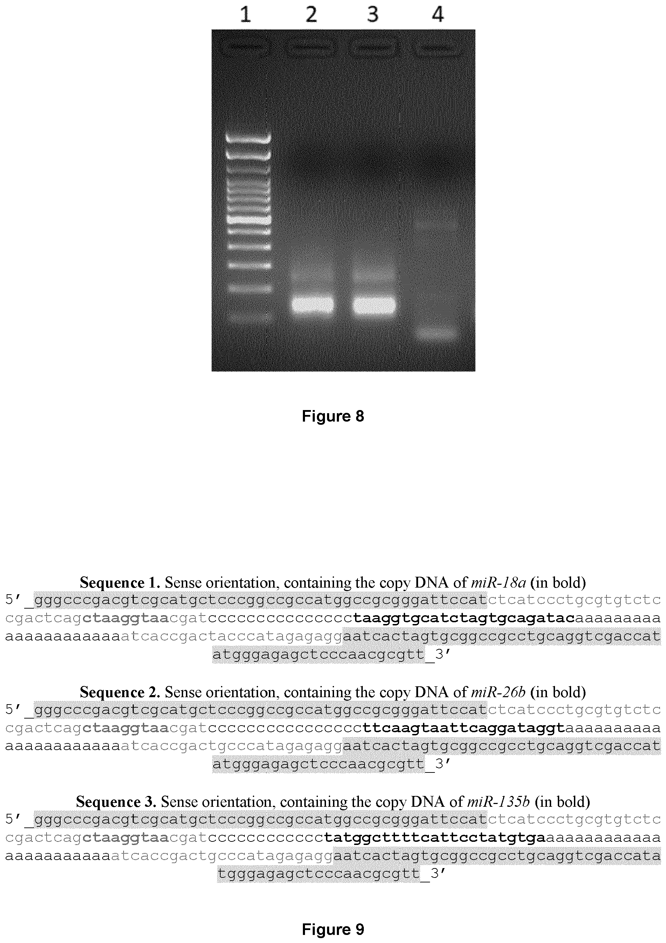

The result is a massive sequencing library with the cDNA of the population of miRNAs flanked by two homopolymers (A/T and G/C) of 10-20 base pairs each. Figure 8 shows the sequencing performed during proof of concept testing.

It should be noted that although the present invention preferably relates to microRNAs (miRNAs), given that they are the most abundant population among small non-coding RNAs, there are other types of small non-coding RNAs such as siRNAs, piwi-RNAs, or tRNAs which can also be detected and quantified by means of the present technology.

In this sense and as it is used throughout the present invention, the term "small non-coding RNAs" encompasses, but is not limited to, a polynucleotide molecule varying from about 10 (preferably 17) to about 450 nucleotides in length, which can be endogenously transcribed or exogenously produced (in a chemical or synthetic manner), but which is not translated into a protein. Preferably, said term encompasses, but is not limited to, a polynucleotide molecule varying from about 10 nucleotides, preferably 15 nucleotides, more preferably 17 nucleotides to about 50 nucleotides, more preferably 30 nucleotides, even more preferably 25 nucleotides in length, which can be endogenously transcribed or exogenously produced (in a chemical or synthetic manner), but which is not translated into a protein.

Examples of small non-coding RNAs include various molecules such as siRNA (small interfering RNA), piwi-RNA, tRNA (transfer ribonucleic acid), snRNA (small nuclear RNA), snoRNA (small nucleolar RNAs), tncRNAs (transfer RNA-derived small ncRNAs), and microRNAs. Likewise, this term, "small non-coding RNAs", also includes primary miRNA transcripts (also known as pri-pre-miRNAS, pri-mirs, and pri-miRNAS) varying from about 70 nucleotides to about 450 nucleotides in length), as well as pre-miRNAS (also known as miRNA precursors, ranging from about 50 nucleotides to about 110 nucleotides in length). In other words, the first aspect of the present invention, as well as all the preferred embodiments of this aspect, can be applied to a method for obtaining a massive sequencing library with the complementary DNA, cDNA, of a population of small non-coding RNAs of interest, which comprises:
a) Capturing the small non-coding RNAs of interest on magnetic particles attached to oligonucleotides bearing in their 3' half a purine polynucleotide sequence of between 18 and 20 bases, preferably poly(T) of between 18 and 20 thymines, and in their 5' half the sequence of one of the massive sequencing adapters, the oligonucleotides being attached to the surface of the magnetic particles by means of a covalent bond at the 5' end thereof, by means of a process which comprises treating the population of small non-coding RNAs with a poly(purine) polymerase, preferably poly(A) polymerase, such that they acquire a 3' end of between 20 and 30 adenine nucleotides (RNA tailing);
b) Carrying out a reverse transcription reaction;
c) Performing alkaline washing on the particles to remove the substrates of the reverse transcription reaction (dehybridizing the small non-coding RNAs and leaving the complementary sequence thereof at the ends of the oligonucleotides) and removing any nucleic acid molecule not covalently attached to the magnetic particles;
d) Blocking the oligonucleotides (attached to the magnetic particles) which have not acquired a cDNA sequence at the 3' end by adding a terminator nucleotide;
e) Performing an elongation of the DNA attached to the particles of the 3' end with a nucleotide tail, preferably a guanine (polyG) tail;
f) Elongating the second massive sequencing adapter in the nucleotide tail, preferably polyG tail, by means of an elongation template consisting of:
   - at the 5' end, a tail of 20 cytosines;
   - at the opposite end, a sequence complementary to the second massive sequencing adapter;
   with the sequence of the small non-coding RNAs of interest being arranged between the two adapters;
g) Performing a polymerization reaction of the DNA, preferably a standard PCR using primers specific for the ends of the two adapters.

Step (a) consists of the specific capturing of genetic material on the surface of the colloidal tools.

In a preferred embodiment, the small non-coding RNAs are selected from any polynucleotide molecule which has a length from 10 nucleotides, more preferably 17 nucleotides, to about 450 nucleotides, and can be endogenously transcribed or exogenously produced (in a chemical or synthetic manner) but cannot be translated into a protein. Preferably, the small non-coding RNAs are selected from any polynucleotide molecule which has a length from about 10 nucleotides, preferably 15 nucleotides, more preferably 17 nucleotides to about 50 nucleotides, more preferably 30 nucleotides, even more preferably 25 nucleotides in length, and can be endogenously transcribed or exogenously produced (in a chemical or synthetic manner) but cannot be translated into a protein.

In another preferred embodiment, the small non-coding RNAs are selected from the list consisting of siRNA (small interfering RNA), piwi-RNA, tRNA (transfer ribonucleic acid or transfer RNA), snRNA (small nuclear RNA), snoRNA (small nucleolar RNA), tncRNAs (transfer RNA-derived small non-coding RNAs), and microRNAs.

In yet another preferred embodiment, the population of small non-coding RNAs of interest comprises non-coding RNAs selected from at least one from the list consisting of siRNAs (small interfering RNAs), piwi-RNAs, tRNAs (transfer ribonucleic acids), snRNAs (small nuclear RNAs), snoRNAs (small nucleolar RNAs), tncRNAs (transfer RNA-derived small ncRNAs), and microRNAs. Preferably, the small non-coding RNAs of interest comprise microRNAs or comprise mainly microRNAs, more specifically more than 80%, 85%, 90%, 95%, 96%, 97%, 98%, or 99% of the total population of small non-coding RNAs are miRNAs. The same proportions could be extrapolated to the other small non-coding RNAs described herein.

### KIT OR DEVICE OF THE INVENTION

Another aspect of the invention relates to a kit or device, hereinafter kit or device of the invention, comprising the elements necessary for carrying out the method of the invention.

### USES OF THE INVENTION

Another aspect of the invention relates to the use of the method of the invention or of the kit or device of the invention for the high-resolution analysis of the populations of miRNAs in a biological sample, and also for the high-resolution analysis of the populations of any other small non-coding RNA such as siRNAs (small interfering RNAs), piwi-RNAs, tRNAs (transfer ribonucleic acids or transfer RNAs), snRNAs (small nuclear RNAs), snoRNAs (small nucleolar RNAs), or tncRNAs (transfer RNA-derived small non-coding RNAs). Preferably, the biological sample is blood, and even more preferably plasma.

The most effective and least costly analysis of miRNAs, as well as of any other small non-coding RNA in blood would have several applications for the diagnosis of various diseases. For example, but without limitation, it would allow the use of circulating miRNAs as a biomarkers for cancer, and more specifically, breast cancer.

Therefore, another aspect of the invention relates to the use of the method of the invention or of the kit or device of the invention for the diagnosis of a disease. More preferably, the disease is cancer. In a particular embodiment, the disease is breast cancer.

### Definitions

Nucleic acids or polynucleotides for sequencing include, but are not limited to, nucleic acids such as DNA, RNA, or PNA (peptide nucleic acid), variants or fragments thereof, and/or concatemers thereof. The polynucleotides can be of a known or unknown sequence, natural or artificial, and can be from any source (for example, eukaryotes or prokaryotes). The polynucleotides can be naturally derived, recombinantly produced, or chemically synthesized. Concatemerized polynucleotides can contain subunits or analogs thereof which may or may not be naturally occurring, or modified subunits. Methods as described herein can be used to determine a polynucleotide sequence. The length of the target nucleic acid for sequencing may vary. For example, the nucleic acid for sequencing can include at least 10, at least 20, at least 30, at least 40, at least 50, at least 100, at least 200, at least 500, at least 1000, at least 10000, at least 100,000, at least 1,000,000, at least 10,000,000 nucleotides. The polynucleotide for sequencing can be of genomic origin or can be fragments or variants thereof. The nucleic acid chain for sequencing can be of single chain and it may or may not be derived from a double-stranded nucleic acid molecule. The single-stranded molecules can also be produced, for example, by means of *in vitro* or chemical synthesis methods and technologies. The embodiments as described in the present specification are not limited by the nucleic acid preparation methods and those skilled in the art can practice any number of methods to provide a composition for use in the described methods. For example, in the sequence by means of synthesis methodologies, a library comprising the target nucleic acids is often generated, and a part of the DNA library is then sequenced.

"Operatively attached" means that two chemical structures are attached to one another such that they remain attached through various manipulations to which they are expected to be subjected. Normally, the functional moiety and the coding oligonucleotide are covalently attached through a suitable binding group. For example, the binding group can be a bifunctional moiety with a binding site for the coding oligonucleotide and a binding site for the functional moiety.

Attachment between the 5' end of the oligonucleotide and the surface of the particle must be by means of a covalent bond. Preferably, there are two options: an amide bond (as shown in the examples of the invention) or bonds based on thiol groups such as a disulfide bond.

The methods described in the present specification are not limited by any sequencing sample preparation method in particular and the alternatives will be readily evident for any person skilled in the art and are considered within of the scope of the present description.

In this specification, the term "colloidal tool" is synonymous to "magnetic particles attached to oligonucleotides".

### EXAMPLES OF THE INVENTION

### EXAMPLE 1

To analyze the working of the methodology, miRNA-seq libraries were produced from synthetic RNA (a collection of 10 synthetic molecules corresponding to human miRNA sequences are used in the test) at very low concentrations (of the order of pmoles and fmoles, in an attempt to emulate existing concentrations.

After completing the miRNA libraries, a proof of concept test was performed by means of Sanger sequencing. The miRNA library is made up of a collection of different sequences, so it is not a suitable substrate for Sanger sequencing.

Purification and ligation of the miRNA libraries in the pGEM plasmid-T (commercial amplicon cloning system) are performed, followed by transformation to *E. coli* (JM109 strain). Then, the bacteria were grown in a selective medium with ampicillin (the pGEM plasmid provides resistance to ampicillin). Only those bacterial clones which took up a plasmid were capable of growing in the selective medium and forming colonies in the agar with the culture medium (the colonies are clones carrying the pGEM plasmid-T with a single insert version).

After cloning, twenty colonies were picked and grown, from which plasmid DNA was extracted, and this was sequenced by means of the Sanger method using specific primers flanking the insertion point. The sequence corresponding to the inserts had a mean size of 128 base pairs.

The insert is attached to the open plasmid by means of a ligation reaction that, if unbiased, would have two options with equal frequency (sense or antisense, with respect to the plasmid sequence). However, in all the sequencing performed in the test of the present invention, the insert was in the sense direction. This datum proves the existence of strong ligation biases which increase when working with relatively short sequences.

Next (Figure 9), 3 examples of Sanger sequencing performed in the proof of concept test are shown, in which the following is observed:
**Sequence 1**. Sense orientation, containing the copy DNA of miR-18a (in bold)
**Sequence 2.** Sense orientation, containing the copy DNA of miR-26b (in bold)
**Sequence 3.** Sense orientation, containing the copy DNA of miR-135b (in bold)
   - Sequences from the pGEM plasmid which contains a miRNA-seq library inserted in its polylinker site are shown shaded.
   - Sequences of the two massive sequencing adapters of the Ion S5 platform are shown in blue:
      ∘ ctcatccctgcgtgtctccgactcagctaaggtaacgat - Adapter A in the sense strand (contains barcode-1 ctaaggtaa). Massive sequencing would commence from the barcode.
      ∘ atcaccgactgcccatagagagg - Adapter TcP1 in the anti-sense strand.
   - Synthetic miRNA cDNA is shown in bold.

It is furthermore observed that the poly(C/G) and poly(A/T) tails have variable sizes due to the actual nature of the tailing reactions and the elongation process on the particle.
**Sequence 1**. Sense orientation, containing the copy DNA of miR-18a (in bold) 
5'_gggcccgacgtcgcatgctcccggccgccatggccgcgggattccatctcatccctgcgtgtctccgactcagctaaggtaa
cgatcccccccccccccctaaggtgcatctagtgcagatacaaaaaaaaaaaaaaaaaatcaccgactacccata
gagaggaatcactagtgcggccgcctgcaggtcgaccatatgggagagctcccaacgcgtt_3'
**Sequence 2.** Sense orientation, containing the copy DNA of miR-26b (in bold) 
5'_gggcccgacgtcgcatgctcccggccgccatggccgcgggattccatctcatccctgcgtgtctccgactcagctaaggtaa
cgatcccccccccccccccttcaagtaattcaggataggtaaaaaaaaaaaaaaaaaaaatcaccgactgcccata
gagaggaatcactagtgcggccgcctgcaggtcgaccatatgggagagctcccaacgcgtt_3'
**Sequence 3.** Sense orientation, containing the copy DNA of miR-135b (in bold)

5'_gggcccgacgtcgcatgctcccggccgccatggccgcgggattccatctcatccctgcgtgtctccgactcagctaaggtaa
cgatcccccccccccctatggcttttcattcctatgtgaaaaaaaaaaaaaaaaaaaaatcaccgactgcccatagag
aggaatcactagtgcggccgcctgcaggtcgaccatatgggagagctcccaacgcgtt_3'

### EXAMPLE 2. Validation of the method by means of analyzing circulating miRNA as a biomarker for breast cancer.

The study consists of two cohorts:
- A cohort of 200 women with breast cancer from whom serum samples will be collected prospectively in Hospital San Cecilio in Granada and in Complejo Hospitalario in Jaén. The samples will be preserved and managed by the Andalusian Biobank (documents of availability and transfer of samples by Biobank are attached). In the event of the existence of neoadjuvant therapy prior to surgery, serum samples will be obtained before and after treatment.
- A control cohort of 30 healthy women without metabolic syndrome and without the presence of cancer in their clinical history. The control cohort should be similar in age to the breast cancer patient cohort. The samples are collected retrospectively and cryopreserved in the Andalusian Biobank.

The study includes control samples of healthy women and sick women with breast cancer in different stages of progression (stages 0, I, II, III, and IV) and with different phenotypes (Luminal A, Luminal B, HER2, and triple negative). In those women undergoing neoadjuvant therapy, the cohort will consist of blood samples obtained before and after treatment.

The high-resolution composition of circulating miRNAs will be evaluated by analyzing the following parameters:
- Ability to identify the presence of breast cancer.
- Ability to identify the different stages of progression of breast cancer.
- Ability to identify the main phenotypes of breast cancer and their correlation with the expression of the main clinical phenotype markers: ER (Estrogen Receptor), PR (Progesterone Receptor), HER2 (Human Epidermal Growth Factor Receptor 2), and Ki67 (nuclear proliferation marker).
- Ability to give a prognosis concerning cancer recurrence, survival, and eradication. This parameter will take into account the evolution of the populations of circulating miRNAs after neoadjuvant therapy.

### EXAMPLE 3. Adaptation to Illumina.

The illustrations shown in the specification show the production of a miRNA library using adapters of the Ion S5 platform. The methodology described in the illustrations can be adapted to the production of libraries for the Illumina platform by introducing small modifications. Taking into account that the length of Illumina adapters is greater than the length of Ion S5 adapters, it is advisable for the reverse transcription and elongation reactions (on particle) to incorporate incomplete versions of Illumina adapters, and for the primers used in final PCR reaction to complete said adapters during the amplification of the library.
- Dispersion of synthetic miRNA pattern massive sequencing data.

Experiments have been performed with synthetic patterns prepared from equimolar mixtures of synthetic RNA sequences corresponding to 30 human miRNAs (miR-17, -18a, -20a, -21, - 23a, -23b, -24, -26b, -29c, -34a, -34b, -34c, -125b, -135a, -135b, -145, -320a, -125a, -130a, - 135b, -150, -155, -200c, -210, -221, -223, -301a, -365a, -454, -663b). The libraries produced with synthetic patterns were massively sequenced in the Illumina platform, incorporating Nextera type adapters. Data analysis disclosed a dispersion of less than 5%, with respect to the equimolarity existing in the initial pattern mixture.
- Sensitivity threshold.

The studies performed with synthetic miRNA patterns have demonstrated that the minimum amount of miRNA which can be used to produce a massive sequencing library is 1 pg.

When libraries are produced from very small amounts of miRNA, relevant increases in amplification bias occur at the PCR level (the invention includes a final PCR step). In these cases, it is advisable to incorporate UMI sequences (Unique Molecular Identifiers, Nat Methods, 2017. PMID: 28448070) into the PCR primers. UMI sequences are used to correct amplification biases.

### CLAUSES

1. A method for obtaining a massive sequencing library with the cDNA of a population of miRNAs of interest, which comprises:
   a) Capturing the miRNAs of interest on magnetic particles attached to oligonucleotides bearing in their 3' half a poly(T) sequence of between 18 and 20 thymines, and in their 5' half the sequence of one of the massive sequencing adapters, the oligonucleotides being attached to the surface of the magnetic particles by means of a covalent bond at the 5' end thereof, by means of a process which comprises treating the population of miRNAs with a poly(A) polymerase, such that they acquire a 3' end of between 20 and 30 adenine nucleotides (RNA tailing);
   b) Carrying out a reverse transcription reaction;
   c) Performing alkaline washing on the particles to remove the substrates of the reverse transcription reaction (dehybridizing the miRNAs and leaving the complementary sequence thereof at the ends of the oligonucleotides) and removing any nucleic acid molecule not covalently attached to the particles;
   d) Blocking the oligonucleotides which have not acquired a cDNA sequence at the 3' end by adding a terminator nucleotide;
   e) Performing an elongation of the DNA attached to the particles of the 3' end with a guanine (polyG) tail;
   f) Elongating the second massive sequencing adapter at the polyG tail by means of an elongation template consisting of:
      - at the 5' end, a tail of 20 cytosines;
      - at the opposite end, a sequence complementary to the second massive sequencing adapter;
      with the sequence of the miRNAs of interest being arranged between the two adapters;
   g) Performing a standard PCR using primers specific for the ends of the two adapters.
2. The method according to clause 1, wherein precipitation and washing with a suitable buffer are performed after each step.
3. The method according to any of clauses 1-2, wherein the elongation of step 5 is performed with a terminal transferase.
4. The method according to any of clauses 1-3, wherein the polyG tail of step 5 must have between 15 and 20 guanine nucleotides.
5. The method according to any of clauses 1-4, wherein the elongation template of step f) can have a dideoxynucleotide at the 3' end which prevents the elongation of the template itself.
6. The method according to any of clauses 1-5, wherein, in the case of libraries produced from tissue RNA in which larger RNAs (ribosomal and messenger RNAs) have not been removed, it is necessary to perform a selective purification of sizes (below 200 bp) after step g).
7. The method according to any of clauses 1-6, wherein other complementary nucleotide ends are used.
8. Use of the method according to any of clauses 1-7 for the identification of miRNAs of interest in a biological sample.
9. Use of the method according to the preceding clause, wherein the biological sample is blood.
10. Use of the method according to clause 8, wherein the biological sample is plasma.
11. Use of the method according to any of clauses 1-7 for the diagnosis, prognosis, or response to treatment of a disease.
12. Use of the method according to any of clauses 1-7 for the diagnosis, prognosis, or response to treatment of cancer.

## Claims

1. A method for obtaining a massive sequencing library with the cDNA of a population of small non-coding RNAs of interest, **characterized in that** said population of small non-coding RNAs are comprised of polynucleotide molecules ranging from 15 nucleotides to 50 nucleotides in length, which can be endogenously transcribed or exogenously produced (in a chemical or synthetic manner) but which are not translated into a protein, wherein the method comprises:
a) Capturing the small non-coding RNAs of interest on magnetic particles attached to oligonucleotides bearing in their 3' half a poly(T) sequence of between 18 and 20 thymines, and in their 5' half the sequence of one of the massive sequencing adapters, the oligonucleotides being attached to the surface of the magnetic particles by means of a covalent bond at the 5' end thereof, by means of a process which comprises treating the population of miRNAs with a poly(A) polymerase, such that they acquire a 3' end of between 20 and 30 adenine nucleotides (RNA tailing);
b) Carrying out a reverse transcription reaction;
c) Performing alkaline washing on the particles to remove the substrates of the reverse transcription reaction (dehybridizing the small non-coding RNAs of interest and leaving the complementary sequence thereof at the ends of the oligonucleotides) and removing any nucleic acid molecule not covalently attached to the particles;
d) Blocking the oligonucleotides which have not acquired a cDNA sequence at the 3' end by adding a terminator nucleotide;
e) Performing an elongation of the DNA attached to the particles of the 3' end with a guanine (polyG) tail;
f) Elongating the second massive sequencing adapter at the polyG tail by means of an elongation template consisting of:
- at the 5' end, a tail of 20 cytosines;
- at the opposite end, a sequence complementary to the second massive sequencing adapter;
with the sequence of the small non-coding RNAs of interest being arranged between the two adapters;
g) Performing a standard PCR using primers specific for the ends of the two adapters.

2. The method according to claim 1, wherein the population of small non-coding RNAs of interest comprises non-coding RNAs selected from at least one from the list consisting of siRNAs (small interfering RNAs), piwi-RNAs, tRNAs (transfer ribonucleic acids), snRNAs (small nuclear RNAs), snoRNAs (small nucleolar RNAs), tncRNAs (transfer RNA-derived small ncRNAs), and microRNAs.

3. The method according to claim 1, wherein the small non-coding RNAs of interest comprise microRNAs.

4. The method according to any of claims 1-3, wherein precipitation and washing with a suitable buffer are performed after each step.

5. The method according to any of claims 1-4, wherein the elongation of step 5 is performed with a terminal transferase.

6. The method according to any of claims 1-5, wherein the polyG tail of step 5 must have between 15 and 20 guanine nucleotides.

7. The method according to any of claims 1-6, wherein the elongation template of step f) can have a dideoxynucleotide at the 3' end which prevents the elongation of the template itself.

8. The method according to any of claims 1-7, wherein, in the case of libraries produced from tissue RNA in which larger RNAs (ribosomal and messenger RNAs) have not been removed, it is necessary to perform a selective purification of sizes (below 200 bp) after step g).

9. The method according to any of claims 1-8, wherein other complementary nucleotide ends are used.

10. Use of the method according to any of claims 1-9 for the identification of miRNAs of interest in a biological sample.

11. Use of the method according to the preceding claim, wherein the biological sample is blood.

12. Use of the method according to claim 10, wherein the biological sample is plasma.

13. Use of the method according to any of claims 1-9 for the diagnosis, prognosis, or response to treatment of a disease.

14. Use of the method according to any of claims 1-9 for the diagnosis, prognosis, or response to treatment of cancer.
